# EUROPEAN PATENT APPLICATION

(11) **EP 2 992 884 A1**
(43) Date of publication of application: **09.03.2016**
(21) Application number: 13883459.3
(22) Date of filing: 08.05.2013
(51) Int. Cl.: A61K 31/496, A61P 31/04, A23K 20/195

(54) **USE OF ENROFLOXACIN SALT IN PREPARING SWINE ORAL PREPARATION**

(30) Priority: 03.05.2013 CN 201310160828
(71) Applicant: Guangzhou Insighter Biotechnology Co., Ltd, Guangzhou, Guangdong 510663 (CN)
(72) Inventor: PENG, Xianfeng, Guangzhou Guangdong 510663 (CN); QIN, Zonghua, Guangzhou Guangdong 510663 (CN)
(74) Representative: Walsh, Marie Goretti
(86) International application number: PCT/CN2013/075325
(87) International publication number: WO 2014/176795

(57) **Abstract**

The invention discloses application of an enrofloxacin salt in preparation of an oral preparation for pigs. The enrofloxacin salt is at least one of salts formed by enrofloxacin and metal ions, and the metal ions are zinc, copper, calcium, magnesium, iron, cobalt, manganese, chromium, silver or nickel. The enrofloxacin salt formed by the enrofloxacin and the metal ions can significantly improve the palatability of the enrofloxacin to pigs; furthermore pharmacokinetics and artificial infection prove that the enrofloxacin salt formed by the enrofloxacin and the metal ions does not affect bioavailability, so enrofloxacin salt can be developed into the oral preparation administrated via the digestive tract, for pig-raising without affecting normal feed intake of the pigs.

## Description

### Field of the Invention

The invention belongs to the field of livestock and poultry breeding and specifically relates to application of an enrofloxacin salt in preparation of an oral preparation for pigs.

### Background of the Invention

Enrofloxacin is the first special quinolone type antibiotic species for animals, has a broad-spectrum bactericidal effect and is effective to bacteria in a stationary phase and a growth phase. The enrofloxacin has a good antibacterial effect on a variety of gram-negative bacilli and cocci, and the MIC against the overwhelming majority of strains of sensitive bacteria is lower than 1ug/ml. The enrofloxacin is widely applied in the aspect of treatment of animal bacterial infections. At present, the forms of the enrofloxacin in commercial products include enrofloxacin base, enrofloxacin sodium and enrofloxacin hydrochloride. It is found in the production practices that the enrofloxacin salt in the three forms has very poor palatability to the pigs and can not be orally administered or administered by mixing, possibly due to the special physiological basis of the pigs. Thus, the enrofloxacin has long been only limited to injections in the application in pig-raising industry.

### Summary of the Invention

An object of the invention is providing application of an enrofloxacin salt in preparation of an oral preparation for pigs.

In the testing process, the inventors found that salts formed by enrofloxacin and part of monovalent (Ag⁺ etc.) or divalent (Zn²⁺ or Cu²⁺ etc.) metal ions could significantly improve the palatability of the enrofloxacin, and could be developed into a preparation administered via the digestive tract, for pig-raising production without affecting the normal feed intake of the pigs. In addition, pharmacokinetics and artificial infection test prove that the salt formed by the enrofloxacin and the metal ions does not affect bioavailability.

Thus, the invention provides application of an enrofloxacin salt in preparation of an oral preparation for pigs, wherein the enrofloxacin salt is at least one of salts formed by enrofloxacin and metal ions, and the metal ions are zinc, copper, calcium, magnesium, iron, cobalt, manganese, chromium, silver or nickel.

The enrofloxacin salt is further preferably enrofloxacin zinc, enrofloxacin copper or enrofloxacin silver.

Preferably, the enrofloxacin zinc is a complex formed by the enrofloxacin and zinc ions according to the molar ratio of 2:1, the enrofloxacin copper is a complex formed by the enrofloxacin and copper ions respectively according to the molar ratio of 2:1, and the enrofloxacin silver is a salt formed by the enrofloxacin and silver ions according to the molar ratio of 1:1.

The oral preparation includes the oral preparation which is orally given or administered via feed or drinking water, and including, e.g., powder, granules, suspensions, emulsions, tablets and pills, etc.

The invention also provides an oral preparation for pigs containing an enrofloxacin salt as an active ingredient, wherein the enrofloxacin salt is at least one of salts formed by enrofloxacin and metal ions, and the metal ions are zinc, copper, calcium, magnesium, iron, cobalt, manganese, chromium, silver or nickel.

The enrofloxacin salt is further preferably enrofloxacin zinc, enrofloxacin copper or enrofloxacin silver.

Preferably, the enrofloxacin zinc is a complex formed by the enrofloxacin and zinc ions according to the molar ratio of 2:1, the enrofloxacin copper is a complex formed by the enrofloxacin and copper ions respectively according to the molar ratio of 2:1, and the enrofloxacin silver is a salt formed by the enrofloxacin and silver ions according to the molar ratio of 1:1.

The oral preparation includes the oral preparation which is orally given or administered via feed or drinking water, and including, e.g., powder, granules, suspensions, emulsions, tablets and pills, etc.

The enrofloxacin salt formed by the enrofloxacin and the metal ions in the invention can significantly improve the palatability of the enrofloxacin to pigs. Furthermore, pharmacokinetics study and artificial infection test prove that the enrofloxacin salt formed by the enrofloxacin and the metal ions does not affect bioavailability. As a result, the enrofloxacin salt can be developed into an oral preparation administrated via the digestive tract, for pig-raising without affecting normal feed intake of the pigs.

### Detailed Description of the Embodiments

The following embodiments are used for further describing the invention rather than limiting the invention.

### Embodiment 1. Preparation of enrofloxacin salt

### 1. Preparation of enrofloxacin zinc

The general formula for the preparation of the enrofloxacin zinc is as follows. 100.0g of enrofloxacin sodium was dissolved in water and the volume was set to 1000mL to obtain an enrofloxacin sodium solution.40.0g of zinc sulfate heptahydrate was dissolved in water and the volume was set to 1000ml to obtain a zinc sulfate solution. The zinc sulfate solution was slowly added dropwise into the enrofloxacin sodium solution at room-temperature under stirring for reaction, and the stirring for reaction was continuously performed for 1h after the end of adding to complete reaction. A suction filtration was performed on the reaction solution, a suction filtration and washing were performed on the filter cake with 3000ml of water, and the filtration residues were dried at 80°C till constant weight so as to obtain the enrofloxacin zinc. 99.30g of sample was produced, and the reaction yield was 96.9%.

2C₁₉H₂₁FN₃O₃Na+ZnSO₄·7H₂O=(C₁₉H₂₁FN₃O₃)₂Zn+Na₂SO₄+7H₂O

### 2. Preparation of enrofloxacin copper

The general formula for the preparation of the enrofloxacin copper is as follows. 100.0g of enrofloxacin sodium was dissolved in water and the volume was set to 1000mL to obtain an enrofloxacin sodium solution. 40.0g of copper sulfate pentahydrate was dissolved in water and the volume was set to 1000ml to obtain a copper sulfate solution. The copper sulfate solution was slowly added dropwise into the enrofloxacin sodium solution at room-temperature under stirring for reaction, and the stirring for reaction was continuously performed for 1h after the end of adding to complete reaction. A suction filtration was performed on the reaction solution, a suction filtration and washing were performed on the filter cake with 3000ml of water, and the filtration residues were dried at 80°C till constant weight so as to obtain the enrofloxacin copper. 98.2g of sample was produced, and the reaction yield was 95.93%.

2C₁₉H₂₁FN₃O₃Na+CuSO₄-5H₂O=(C₁₉H₂₁FN₃O₃)₂Cu+Na₂SO₄+5H₂O

### 3. Preparation of enrofloxacin silver

The general formula for the preparation of the enrofloxacin silver is as follows. 100.0g of enrofloxacin sodium was dissolved in water and the volume was set to 1000mL to obtain an enrofloxacin sodium solution. 50.0g of silver nitrate was dissolved in water and the volume was set to 1000ml to obtain a silver nitrate solution. The silver nitrate solution was slowly added dropwise into the enrofloxacin sodium solution at room-temperature under stirring for reaction, and the stirring for reaction was continuously performed for 1h after the end of adding to complete reaction. A suction filtration was performed on the reaction solution, a suction filtration and washing were performed on the filter cake with 3000ml of water, and filtration residues were dried at 80°C till constant weight so as to obtain the enrofloxacin silver. 126.6g of sample was produced, and the reaction yield was 96.89%.

C₁₉H₂₁FN_{3O3}Na+AgNO₃=C₁₉H₂₁FN₃O₃Ag+NaNO₃

### Embodiment 2. Influence of enrofloxacin base, sodium salt and hydrochloride on palatability of pig feed

100 105-day-old healthy duroc-landrace-yorkshire hybrid pigs with similar body weights were divided into 10 test groups as shown in Table 1, and each group comprised 10 pigs. Enrofloxacin base, enrofloxacin sodium and enrofloxacin hydrochloride were respectively added in feed in each group. All the test pigs were synchronously fasted for 12h before testing. The test pigs in each group were respectively subjected to free feed intake of feed containing enrofloxacin in different forms after the 12h fast. Observation was performed for 30-60 minutes till the test pigs did not take feed any more. Feed consumption of different test groups was subjected to statistics and the influence of different forms of the enrofloxacin added in the feed on the feed intake of the pigs was evaluated. The pure feed which was not added with the enrofloxacin or the salt thereof was taken as control.

**Table 1: Test grouping of influence of enrofloxacin base, enrofloxacin sodium or enrofloxacin hydrochloride on palatability of pig feed**

| Group | Test sample | Dose* (ppm) | Number of test pigs |
|---|---|---|---|
| 1 | enrofloxacin base | 5 | 10 |
| 2 | enrofloxacin base | 25 | 10 |
| 3 | enrofloxacin base | 100 | 10 |
| 4 | enrofloxacin sodium | 5 | 10 |
| 5 | enrofloxacin sodium | 25 | 10 |
| 6 | enrofloxacin sodium | 100 | 10 |
| 7 | enrofloxacin hydrochloride | 5 | 10 |
| 8 | enrofloxacin hydrochloride | 25 | 10 |
| 9 | enrofloxacin hydrochloride | 100 | 10 |
| 10 | control group (-) | | 10 |

| | | | |
|---|---|---|---|
| *: All the test doses were calculated by enrofloxacin. | | | |

As the enrofloxacin base, the enrofloxacin sodium or the enrofloxacin hydrochloride was added in the feed, the feed intake of each test pig was significantly reduced. Comparing with a blank control group, the feed intake in each of the test groups added with 5ppm of enrofloxacin base, enrofloxacin sodium or enrofloxacin hydrochloride respectively was reduced by 47.2%, 50.8% and 49.5% respectively, and the test pigs in the group added with 100ppm of the enrofloxacin in different forms showed apastia (See table 2 for the results).

**Table 2: Test results of influence of enrofloxacin and salts thereof on palatability of pig feed**

| Group | Using amount (ppm) | Dose (ppm) | Total feed intake (kg) | Average feed intake (kg) | Decline in feed intake (%) |
|---|---|---|---|---|---|
| 1 | enrofloxacin base | 5 | 6.81 | 0.681 | 47.2 |
| 2 | enrofloxacin base | 25 | 3.32 | 0.332 | 74.2 |
| 3 | enrofloxacin base | 100 | 0.86 | 0.086 | 93.3 |
| 4 | enrofloxacin sodium | 5 | 6.34 | 0.634 | 50.8 |
| 5 | enrofloxacin sodium | 25 | 3.47 | 0.347 | 73.1 |
| 6 | enrofloxacin sodium | 100 | 0.76 | 0.076 | 94.1 |
| 7 | enrofloxacin hydrochloride | 5 | 6.51 | 0.651 | 49.5 |
| 8 | enrofloxacin hydrochloride | 25 | 3.41 | 0.341 | 73.4 |
| 9 | enrofloxacin hydrochloride | 100 | 0.81 | 0.081 | 93.7 |
| 10 | control group (-) | - | | 1.289 | - |

### Embodiment 3. Influence of enrofloxacin salts on palatability of pig feed

150 110-day-old healthy duroc-landrace-yorkshire hybrid pigs with similar body weights were divided into 5 test groups as shown in Table 3, and each group performed three repeats. The feed in each group was added with different doses of the enrofloxacin or the salt thereof. All the test pigs were synchronously fasted for 12h before testing. The test pigs in each group were respectively subjected to free feed intake of feed containing enrofloxacin or the salt thereof after the 12h fast. Observation was performed for 30-60 minutes till the test pigs did not take feed any more. Feed consumption of different test groups was subjected to statistics and the influence of different forms of the enrofloxacin on the palatability of pig feed was evaluated. The pure feed which was not added with the enrofloxacin or the salt thereof was taken as control.

**Table 3: Test grouping of influence of enrofloxacin and salts thereof on palatability of pig feed**

| Group | Test sample | Dose* (ppm) | Number of test pigs |
|---|---|---|---|
| 1 | enrofloxacin sodium | 100 | 10×3 |
| 2 | enrofloxacin zinc | 100 | 10×3 |
| 3 | enrofloxacin copper | 100 | 10×3 |
| 4 | enrofloxacin silver | 100 | 10×3 |
| 5 | control group (-) | - | 10×3 |

| | | | |
|---|---|---|---|
| *: All the test doses were calculated by enrofloxacin. | | | |

As the enrofloxacin or the salt was added into the feed, enrofloxacin sodium basically completely inhibited the feed intake behavior of pigs , while salts formed by enrofloxacin and zinc, copper and silver had no significant influence on the feed intake of the test pigs (See table 4 for the results).

**Table 4: Test results of influence of enrofloxacin and salts thereof on palatability of pig feed**

| Group | Test sample (ppm) | Number of animals | Total feed intake (kg) | Average feed intake (kg) |
|---|---|---|---|---|
| 1 | enrofloxacin sodium (100ppm) | 10×3 | 2.18 | 0.073±0.015^{B} |
| 2 | enrofloxacin zinc (100ppm) | 10×3 | 39.55 | 1.318±0.182^{A} |
| 3 | enrofloxacin copper (100ppm) | 10×3 | 38.90 | 1.297±0.203^{A} |
| 4 | enrofloxacin silver (100ppm) | 10×3 | 38.72 | 1.291±0.180^{A} |
| 5 | control group (-) | 10×3 | 39.01 | 1.300±0.166^{A} |

| | | | | |
|---|---|---|---|---|
| Note: Data in the same column with different capital letters as superscripts showed significant difference (P<0.05). | | | | |

### Embodiment 4. Dose research of influence of enrofloxacin zinc on palatability of pig feed

120 110-day-old healthy duroc-landrace-yorkshire hybrid pigs with similar body weights were divided into 4 test groups as shown in Table 5, each group performed three repeats, and each repeat was performed on 10 pigs. The feed in each group was added with different doses of the enrofloxacin zinc respectively. All the test pigs were synchronously fasted for 12h before testing. The test pigs in each group were respectively subjected to free feed intake of feed containing enrofloxacin zinc in different forms after the 12h fast. Observation was performed for 30-60 minutes till the test pigs did not take feed any more. Feed consumption of different test groups was subjected to statistics and the influence of different doses of the enrofloxacin zinc added into the feed on palatability of pig feed was evaluated. The pure feed which was not added with the enrofloxacin or the salt thereof was taken as control.

**Table 5: Test grouping of dose research of influence of enrofloxacin zinc on palatability of pig feed**

| Group | Test sample | Dose* (ppm) | Number of test pigs |
|---|---|---|---|
| 1 | enrofloxacin zinc | 500 | 10×3 |
| 2 | enrofloxacin zinc | 250 | 10×3 |
| 3 | enrofloxacin zinc | 100 | 10×3 |
| 4 | control group (-) | - | 10×3 |

| | | | |
|---|---|---|---|
| *: All the test doses were calculated by enrofloxacin. | | | |

As feed was added with different doses of enrofloxacin zinc, the feed intake of the test pigs had no significant difference from that of a control group (the pure feed which was not added with enrofloxacin or the salt thereof) (See table 6 for the results).

**Table 6: Test results of influence of enrofloxacin and salts thereof on palatability of pig feed**

| Group | Test sample | Dose (ppm) | Average feed intake (kg) |
|---|---|---|---|
| 1 | enrofloxacin zinc | 500 | 1.338±0.259^{A} |
| 2 | enrofloxacin zinc | 250 | 1.319±0.297^{A} |
| 3 | enrofloxacin zinc | 100 | 1.335±0.342^{A} |
| 4 | control group (-) | - | 1.324±0.315^{A} |

| | | | |
|---|---|---|---|
| Note: *Data in the same column with different capital letters as superscripts showed significant difference (P<0.05). | | | |

### Embodiment 5. Dose research of influence of enrofloxacin copper on palatability of pig feed

120 115-day-old healthy duroc-landrace-yorkshire hybrid pigs with similar body weights were divided into 4 test groups as shown in Table 7, each group performed three repeats, and each repeat was performed on 10 pigs. The feed in each group was added with different doses of the enrofloxacin copper respectively. All the test pigs were synchronously fasted for 12h before testing. The test pigs in each group were respectively subjected to free feed intake of feed containing enrofloxacin copper in different forms after the 12h fast. Observation was performed for 30-60 minutes till the test pigs did not take feed any more. Feed consumption of different test groups was subjected to statistics and the influence of different doses of the enrofloxacin copper added into the feed on palatability of pig feed was evaluated. The pure feed which was not added with the enrofloxacin or the salt thereof was taken as control.

**Table 7: Test grouping of dose research of influence of enrofloxacin copper on palatability of pig feed**

| Group | Test sample | Dose* (ppm) | Number of test pigs |
|---|---|---|---|
| 1 | enrofloxacin copper | 500 | 10×3 |
| 2 | enrofloxacin copper | 250 | 10×3 |
| 3 | enrofloxacin copper | 100 | 10×3 |
| 4 | control group (-) | - | 10×3 |

| | | | |
|---|---|---|---|
| *: All the test doses were calculated by enrofloxacin. | | | |

As feed was added with different doses of enrofloxacin copper, the feed intake of the test pigs had no significant difference from that of a control group (See table 8 for the results).

**Table 8: Test results of influence of enrofloxacin copper on palatability of pig feed**

| Group | Test sample | Dose (ppm) | Average feed intake (kg) |
|---|---|---|---|
| 1 | enrofloxacin copper | 500 | 1.352±0.351^{A} |
| 2 | enrofloxacin copper | 250 | 1.369±0.349^{A} |
| 3 | enrofloxacin copper | 100 | 1.355±0.397^{A} |
| 4 | control group (-) | - | 1.358±0.381^{A} |

| | | | |
|---|---|---|---|
| Note: *Data in the same column with different capital letters as superscripts showed significant difference (P<0.05). | | | |

### Embodiment 6. Dose research of influence of enrofloxacin silver on palatability of pig feed

120 120-day-old healthy duroc-landrace-yorkshire hybrid pigs with similar body weights were divided into 4 test groups as shown in Table 9, each group performed three repeats, and each repeat was performed on 10 pigs. The feed in each group was added with different doses of the enrofloxacin silver respectively. All the test pigs were synchronously fasted for 12h before testing. The test pigs in each group were respectively subjected to free feed intake of feed containing enrofloxacin silver in different forms after the 12h fast, observation was performed for 30-60 minutes till the test pigs did not take feed any more. Feed consumption of different test groups was subjected to statistics and the influence of different doses of the enrofloxacin silver added into the feed on palatability of pig feed was evaluated. The pure feed which was not added with the enrofloxacin or the salt thereof was taken as control.

**Table 9: Test grouping of dose research of influence of enrofloxacin silver on palatability of pig feed**

| Group | Test sample | Dose* (ppm) | Number of test pigs |
|---|---|---|---|
| 1 | enrofloxacin silver | 500 | 10×3 |
| 2 | enrofloxacin silver | 250 | 10×3 |
| 3 | enrofloxacin silver | 100 | 10×3 |
| 4 | control group (-) | - | 10×3 |

| | | | |
|---|---|---|---|
| *: All the test doses were calculated by enrofloxacin. | | | |

As feed was added with different doses of enrofloxacin silver, the feed intake of the test pigs had no significant difference from that of a control group (See table 10 for results).

**Table 10: Test results of dose research of influence of enrofloxacin silver on palatability of pig feed**

| Group | Test sample | Dose (ppm) | Average feed intake (kg) |
|---|---|---|---|
| 1 | enrofloxacin silver | 500 | 1.379±0.388^{A} |
| 2 | enrofloxacin silver | 250 | 1.389±0.447^{A} |
| 3 | enrofloxacin silver | 100 | 1.380±0.416^{A} |
| 4 | control group (-) | - | 1.385±0.406^{A} |

| | | | |
|---|---|---|---|
| Note: *Data in the same column with different capital letters as superscripts showed significant difference (P<0.05). | | | |

### Embodiment 7. Influence of long-term application of enrofloxacin zinc, enrofloxacin copper and enrofloxacin silver on feed intake of pigs

120 120-day-old healthy duroc-landrace-yorkshire hybrid pigs with similar body weights were divided into 4 test groups as shown in Table 11, and each group performed three repeats. Feed in each group was respectively added with 100ppm of enrofloxacin zinc, enrofloxacin copper or enrofloxacin silver. The feed intake of the test pigs for 7 continuous days was subjected to statistics, the average daily feed intake was calculated, and influence of the normal application of the enrofloxacin zinc and the like on the feed intake of the pigs was evaluated. The pure feed which was not added with the enrofloxacin or the salt thereof was taken as control.

**Table 11: Test grouping of influence of enrofloxacin zinc and the like on feed intake of test pigs**

| Group | Test sample | Dose* (ppm) | Number of test pigs |
|---|---|---|---|
| 1 | enrofloxacin zinc | 100 | 10×3 |
| 2 | enrofloxacin copper | 100 | 10×3 |
| 3 | enrofloxacin silver | 100 | 10×3 |
| 4 | control group (-) | - | 10×3 |

| | | | |
|---|---|---|---|
| *: All the test doses were calculated by enrofloxacin. | | | |

Statistical results of the continuous 7 days showed that as feed was added with enrofloxacin zinc and the like, comparing with a control group which was not added with a medicament, the feed intake behavior and the feed intake of the test pigs were normal, and the feed intake had no significant difference statistically (See table 12 for the results).

**Table 12: Test results of influence of enrofloxacin zinc and the like on feed intake of test pigs**

| Group | Test sample | Number of animals | Total feed intake (kg) | Average feed intake (kg) |
|---|---|---|---|---|
| 1 | enrofloxacin zinc | 10×3 | 468.3 | 2.230±0.321^{A} |
| 2 | enrofloxacin copper | 10×3 | 463.5 | 2.207±0.333^{A} |
| 3 | enrofloxacin silver | 10×3 | 460.1 | 2.191±0.280^{A} |
| 4 | control group (-) | 10×3 | 458.9 | 2.185±0.316^{A} |

| | | | | |
|---|---|---|---|---|
| Note: Data in the same column with different capital letters as superscripts showed significant difference (P<0.05). | | | | |

### Embodiment 8. Pharmacokinetic research of enrofloxacin zinc and the like in pigs

20 40-day-old piglets were divided into four groups with five pigs in each group as shown in Table 13, and the pigs were fed with enrofloxacin in different forms by gastric tubes in an amount of 10mg/kg body weight respectively. Jugular venous blood collection was performed at 30min, 1h, 2h, 4h, 8h, 12h and 24h after administration. Plasma was separated, the concentration of the enrofloxacin in the plasma in the different test pigs after administration was determined by high-performance liquid chromatography respectively, and whether the difference in the concentration of the enrofloxacin in the plasma in each test group after administration was significant or not and whether the elimination rule of the enrofloxacin in blood was consistent or not was subjected to statistical analysis.

**Table 13. Test grouping of pharmacokinetic research of enrofloxacin zinc and the like in pigs**

| Group | Test sample | Dose*(mg/kg of body weight) | Number of test pigs | Administration way |
|---|---|---|---|---|
| 1 | enrofloxacin zinc | 10 | 5 | Administered by gastric tube |
| 2 | enrofloxacin copper | 10 | 5 | Administered by gastric tube |
| 3 | enrofloxacin silver | 10 | 5 | Administered by gastric tube |
| 4 | enrofloxacin sodium | 10 | 5 | Administered by gastric tube |

The concentration and the elimination rule of the enrofloxacin and ciprofloxacin in the plasma of the test pigs in each test group after administration are as shown in Table 14. The results showed that the peak values of the plasma concentrations of all the test groups were similar, and the elimination law was consistent, indicating that the salts formed by the enrofloxacin and metal ions, such as zinc, copper, silver and the like did not affect the absorption and metabolism processes of the enrofloxacin in vivo.

**Table 14: Changing law of plasma concentration of enrofloxacin zinc and the like in pigs (unit: ug/ml)**

| Group | Test sample | 30min | 1h | 2h | 4h | 8h | 12h | 24h |
|---|---|---|---|---|---|---|---|---|
| 1 | enrofloxacin zinc | 1.432± 0.186^{A} | 2.189± 0.204^{B} | 5.321± 0.541^{C} | 5.358± 0.404^{D} | 2.998± 0.216^{E} | 1.762± 0.205^{F} | 0.311± 0.045^{G} |
| 2 | enrofloxacin copper | 1.417± 0.169^{A} | 2.121± 0.198 ^{B} | 5.187± 0.558 ^{C} | 5.465± 0.529 ^{D} | 2.986± 0.234 ^{E} | 1.785± 0.215 ^{F} | 0.294± 0.044^{G} |
| 3 | enrofloxacin silver | 1.422± 0.189 ^{A} | 2.250± 0.256 ^{B} | 5.425± 0.596 ^{C} | 5.518± 0.498 ^{D} | 2.977± 0.231 ^{E} | 1.779± 0.194 ^{F} | 0.308± 0.048^{G} |
| 4 | enrofloxacin sodium | 1.426± 0.191^{A} | 2.230± 0.251 ^{B} | 5.338± 0.555 ^{C} | 5.299± 0.484 ^{D} | 2.987± 0.266 ^{E} | 1.781± 0.167 ^{F} | 0.298± 0.042^{G} |

| | | | | | | | | |
|---|---|---|---|---|---|---|---|---|
| Note: Data in the same column with different capital letters as superscripts showed significant difference (P<0.05); and the numerical value of the plasma concentration in the table was the total amount of the enrofloxacin and the ciprofloxacin. | | | | | | | | |

### Embodiment 9. Test results of enrofloxacin zinc and the like against escherichia coli artificial infection

60 30-day-old weaned piglets were divided into six groups with 10 pigs in each group as shown in Table 15, feed was respectively added with enrofloxacin in different forms, and free feed intake was performed. The 33-day-old pigs were intramuscularly injected with pathogenic escherichia coli, the diarrhea and deaths of the pigs were observed, continuous observation was performed for one week, the onset and deaths were subjected to statistics, and the protection effects of the enrofloxacin in different forms against the pathogenic escherichia coli in the artificial infection were compared. Pure feed which was not added with the enrofloxacin in any form was taken as a control group, wherein one portion of pigs in the control group were intramuscularly injected with the pathogenic escherichia coli and was taken as the control group without administration but with challenge (the fifth group), and the remainder were not intramuscularly injected with the pathogenic escherichia coli and was taken as the control group without administration and without challenge (the sixth group).

**Table 15: Test grouping of protection effects of enrofloxacin in different forms against escherichia coli challenge infection**

| Group | Test sample | Dose* (ppm) | Number of test pigs | Challenge strain | Challenge dose (×107CFU/pig) |
|---|---|---|---|---|---|
| 1 | enrofloxacin zinc | 100 | 10 | escherichia coli SGD strain | 2.0 |
| 2 | enrofloxacin copper | 100 | 10 | escherichia coli SGD strain | 2.0 |
| 3 | enrofloxacin silver | 100 | 10 | escherichia coli SGD strain | 2.0 |
| 4 | enrofloxacin base# | 100 | 10 | escherichia coli SGD strain | 2.0 |
| 5 | - | - | 10 | escherichia coli SGD strain | 2.0 |
| 6 | - | - | 10 | - | - |

| | | | | | |
|---|---|---|---|---|---|
| Note: *All the test doses were calculated by the enrofloxacin; # the administration way of the test group of the enrofloxacin base was that feeding was performed twice through a gastric tube in the morning and the evening daily according to the feed intake of each test pig. | | | | | |

After artificial infection with an escherichia coli SGD strain, all the test pigs in the control group without administration but with challenge (the fifth group) showed diarrhea, wherein 6 pigs died in the test period, while all the test pigs in the control group without the administration and without challenge (the sixth group) grew normally without diarrhea and death. All the medication administration groups showed complete protection effects against escherichia coli artificial infection (See table 16 for the results).

**Table 16: Protection effects of enrofloxacin in different forms against escherichia coli challenge infection**

| Group | Test sample | Number of test pigs | Challenge (Yes/No) | Incidence rate (%, calculated by diarrhea) | Death rate (%) |
|---|---|---|---|---|---|
| 1 | enrofloxacin zinc | 10 | Yes | 0 | 0 |
| 2 | enrofloxacin copper | 10 | Yes | 0 | 0 |
| 3 | enrofloxacin silver | 10 | Yes | 0 | 0 |
| 4 | enrofloxacin base^{#} | 10 | Yes | 0 | 0 |
| 5 | - | 10 | Yes | 100 | 60 |
| 6 | - | 10 | No | 0 | 0 |

## Claims

1. Application of an enrofloxacin salt in preparation of an oral preparation for pigs, wherein the enrofloxacin salt is at least one of salts formed by enrofloxacin and metal ions, and the metal ions are zinc, copper, calcium, magnesium, iron, cobalt, manganese, chromium, silver or nickel.

2. The application according to claim 1, wherein the enrofloxacin salt is enrofloxacin zinc, enrofloxacin copper or enrofloxacin silver.

3. The application according to claim 2, wherein the enrofloxacin zinc is a complex formed by the enrofloxacin and zinc ions according to the molar ratio of 2:1, the enrofloxacin copper is a complex formed by the enrofloxacin and copper ions respectively according to the molar ratio of 2:1, and the enrofloxacin silver is a salt formed by the enrofloxacin and silver ions according to the molar ratio of 1:1.

4. The application according to claim 1 or 2 or 3, wherein the oral preparation includes the oral preparation which is orally given or administered via feed or drinking water.

5. The application according to claim 4, wherein the oral preparation includes powder, granules, suspensions, emulsions, tablets or pills.

6. An oral preparation for pigs, containing an enrofloxacin salt as an active ingredient, wherein the enrofloxacin salt is at least one of salts formed by enrofloxacin and metal ions, and the metal ions are zinc, copper, calcium, magnesium, iron, cobalt, manganese, chromium, silver or nickel.

7. The oral preparation for the pigs according to claim 6, wherein the enrofloxacin salt is enrofloxacin zinc, enrofloxacin copper or enrofloxacin silver.

8. The oral preparation for the pigs according to claim 7, wherein the enrofloxacin zinc is a complex formed by the enrofloxacin and zinc ions according to the molar ratio of 2:1, the enrofloxacin copper is a complex formed by the enrofloxacin and copper ions respectively according to the molar ratio of 2:1, and the enrofloxacin silver is a salt formed by the enrofloxacin and silver ions according to the molar ratio of 1:1.

9. The oral preparation for the pigs according to claim 6 or 7 or 8, wherein the oral preparation includes the oral preparation which is orally given or administered via feed or drinking water.

10. The oral preparation for the pigs according to claim 9, wherein the oral preparation includes powder, granules, suspensions, emulsions, tablets or pills.
